# FASCICULE DE BREVET EUROPEEN

(11) **EP 1 921 448 B1**
(45) Date de publication et mention de la délivrance du brevet: **08.07.2015**
(21) Numéro de dépôt: 07356159.9
(22) Date de dépôt: 12.11.2007
(51) Int. Cl.: G01N 33/18

(54) **Dispositif et installation de prélèvement et de test qualitatif de l'eau d'un bassin, tel qu'une piscine.**
Vorrichtung und Anlage zur Entnahme und Qualitätsprüfung von Wasser aus einem Becken, beispielsweise eines Schwimmbads
Device and facility for sampling and qualitatively testing the water in a basin, such as a swimming pool.

(30) Priorité: 13.11.2006 FR 0609889
(43) Date de publication de la demande: 14.05.2008
(73) Titulaire: Covaltech, 69260 Charbonnières-les-bains (FR)
(72) Inventeur: Selles, Serge Albert Pierre, 69009 Lyon (FR); Angonin, Michel, 69260 Charbonnieres les Bains (FR)
(74) Mandataire: Grand, Guillaume

(56) Documents cités:
- EP-A- 0 848 242
- WO-A-2004/059324
- FR-A1- 2 708 347
- US-A- 3 802 782
- US-A- 5 474 938

## Description

La présente invention concerne un dispositif et une installation de prélèvement et de test qualitatif de l'eau d'un bassin, tel qu'une piscine.

L'entretien d'une piscine, individuelle ou collective, nécessite d'effectuer, à titre régulier, des prélèvements d'eau puis de déterminer des paramètres physico-chimiques de l'eau prélevée, par exemple son alcalinité, c'est-à-dire son titre alcalimétrique complet (TAC). Actuellement, pour ce faire, on mélange une quantité dosée d'eau prélevée avec un ou plusieurs réactifs appropriés, tels que de l'acide adipique et du vert de bromocrésol lorsqu'on cherche à déterminer la concentration en bicarbonate pour quantifier l'alcalinité de l'eau. Par observation de la couleur du mélange aqueux obtenu, voire par mesure de sa densité optique au moyen d'un photomètre, on déduit une valeur au moins approximative du paramètre physico-chimique associé au réactif utilisé. Si cette valeur n'est pas conforme aux exigences sanitaires correspondantes, des actions correctrices de la qualité de l'eau doivent être entreprises.

En pratique, les opérations de prélèvement et de test qualitatif de l'eau d'une piscine se révèlent fastidieuses. La personne chargée de l'entretien doit, au moyen d'une pipette ou, plus généralement, d'un instrument de prélèvement manuel, doser une quantité d'eau à tester, puis introduire cette quantité d'eau dans le compartiment d'un testeur, pour la mélanger à un réactif qui, lui aussi, doit être introduit en quantité dosée, à moins qu'une quantité prédéterminée de ce réactif ne soit préalablement fournit au fond du compartiment. Une fois le mélange réalisé, la personne chargée de l'entretien doit observer attentivement la solution contenue dans le compartiment du testeur, voire l'installer dans un photomètre, pour déterminer une valeur du paramètre physico-chimique. En outre, on comprend que la répétabilité des mesures est médiocre, puisque, selon le soin apporté par la personne pour effectuer le prélèvement de l'eau, le dosage de cette eau et la mesure du paramètre physico-chimique, les résultats souffrent d'une grande dispersion. Cet inconvénient est d'autant plus marqué lorsque l'entretien est réalisé par différentes personnes, comme c'est couramment le cas pour les piscines collectives, avec les risques sanitaires que cela implique.

US-A-5 474 938, sur lequel est basé le préambule de la revendication 1, propose un dispositif de prélèvement et de test de l'eau d'une piscine, dans lequel l'eau est prélevée par une vanne commandée et envoyée dans un compartiment fixe. Dans ce compartiment, des réactifs sont mélangés à l'eau et la réaction physico-chimique alors obtenue est observée par une cellule photoélectrique reliée à un ordinateur. Ce dispositif est plus évolué que l'instrumentation manuelle évoquée ci-dessus, mais s'avère en fait peu pratique dans le sens où, après avoir réalisé un mélange de test dans le compartiment fixe, ce dernier est à rincer avec précaution, faute de quoi les mesures optiques relatives aux mélanges subséquents risquent d'être faussées. Cela pose, entre autres, des problèmes d'évacuation des eaux de rinçage.

De manière plus éloignée à la problématique considérée ici, WO-A-2004/059324 divulgue, de manière peu précise, un dispositif de test d'eau, qui comporte un module-capteur d'une part et un module-détecteur d'autre part. Le module-capteur inclut des capteurs dont une surface est prévue pour détecter la présence ou l'absence d'un agent spécifique, tel qu'un agent pathogène. Le module-détecteur comporte des détecteurs optiques, qui, s'ils sont bien alignés avec les capteurs, observent l'état de surface de ces capteurs. Pour automatiser le fonctionnement de ce dispositif, les capteurs peuvent être entraînés automatiquement depuis une cartouche de capteurs non encore utilisés jusqu'à une cartouche de capteurs usagés, en étant, entre les deux cartouches, plongés individuellement dans un compartiment fixe, rempli d'eau à tester. Ce dispositif n'est donc pas adapté à la mesure des caractéristiques physico-chimiques traditionnellement contrôlées pour les eaux de piscine et la fiabilité des observations par le module-détecteur nécessite une commande en déplacement des capteurs très précise.

Le but de la présente invention est de proposer un dispositif de prélèvement et de test qualitatif de l'eau d'un bassin, qui soit d'utilisation particulièrement aisée et qui garantisse de bonnes précision et répétabilité des mesures des paramètres physico-chimiques de l'eau testée.

A cet effet, l'invention a pour objet un dispositif de prélèvement et de test qualitatif de l'eau d'un bassin, tel qu'une piscine, tel que défini à la revendication 1.

Grâce à l'invention, on dispose d'un dispositif automatique qui, en service, ne nécessite pas d'intervention manuelle en ce qui concerne le prélèvement d'eau du bassin et la mesure des caractéristiques physico-chimiques de cette eau. En effet, les moyens de remplissage de ce dispositif sont automatisés dans le sens où une quantité dosée pré-établie d'eau du bassin est prélevée par ces moyens, avant d'être introduite dans le ou les compartiments de la barrette. De cette façon, excepté au cours du réglage du dispositif, la quantité d'eau prélevée et envoyée à la barrette est constante dans le temps, garantissant la répétabilité des mesures. En outre, en raison de leur fonctionnement automatique, ces moyens de remplissage sont particulièrement pratiques et, en étant associés aux moyens d'entraînement motorisés de la barrette au sein du châssis de support selon l'invention, ils rendent facile l'utilisation du dispositif. En pratique, l'utilisateur du dispositif n'a qu'à s'assurer régulièrement du bon approvisionnement du dispositif en énergie et en barrettes non encore utilisées.

En outre, les moyens de mesure sont appliqués aux solutions aqueuses présentes dans les barrettes successivement testées de manière répétitive, et ce dans les mêmes conditions. Ainsi, même si après un étalonnage initial du dispositif, les mesures réalisées par ces moyens dérivent, les résultats de mesure successifs gardent une certaine cohérence dans le temps, qu'il est possible d'exploiter pour conclure quant à la qualité de l'eau testée. En pratique, pour des raisons de précision, ces moyens de mesure sont avantageusement de nature optique, mais d'autres formes de ces moyens, telles que des sondes, peuvent être utilisées. On comprend que l'entraînement prédéterminé des barrettes au sein du châssis du dispositif, qui constitue en quelque sorte une enceinte pseudo-fermée, participe à la qualité, à la précision et à la cohérence des mesures effectuées.

D'autres caractéristiques avantageuses de ce dispositif, prises isolément ou suivant toutes les combinaisons techniquement possibles, sont énoncées aux revendications 2 à 8.

L'invention a également pour objet une installation de prélèvement et de test qualitatif de l'eau d'un bassin, tel qu'une piscine, telle que définie à la revendication 9.

L'invention sera mieux comprise à la lecture de la description qui va suivre, donnée uniquement à titre d'exemple et faite en se référant aux dessins sur lesquels :
- la figure 1 est un schéma d'une piscine et d'un circuit de circulation d'eau provenant de cette piscine, équipé d'une installation conforme à l'invention ;
- la figure 2 est une perspective schématique de l'installation de la figure 1 ;
- la figure 3 est une vue en perspective d'un dispositif, conforme à l'invention, appartenant à l'installation de la figure 2, certains des composants de ce dispositif n'étant pas représentés pour des raisons de visibilité ;
- la figure 4 est une section schématique selon le plan IV de la figure 3 ;
- les figures 5 et 6 sont des sections schématiques selon la ligne V-V de la figure 4, illustrant respectivement deux états de fonctionnement distincts, différents de celui illustré à la figure 4 ;
- la figure 7 est une vue en élévation selon la flèche VII de la figure 3, avec arrachement partiel ;
- la figure 8 est une coupe schématique du détail cerclé VIII à la figure 7 ; et
- la figure 9 est une vue analogue à la figure 7, illustrant un état de fonctionnement différent.

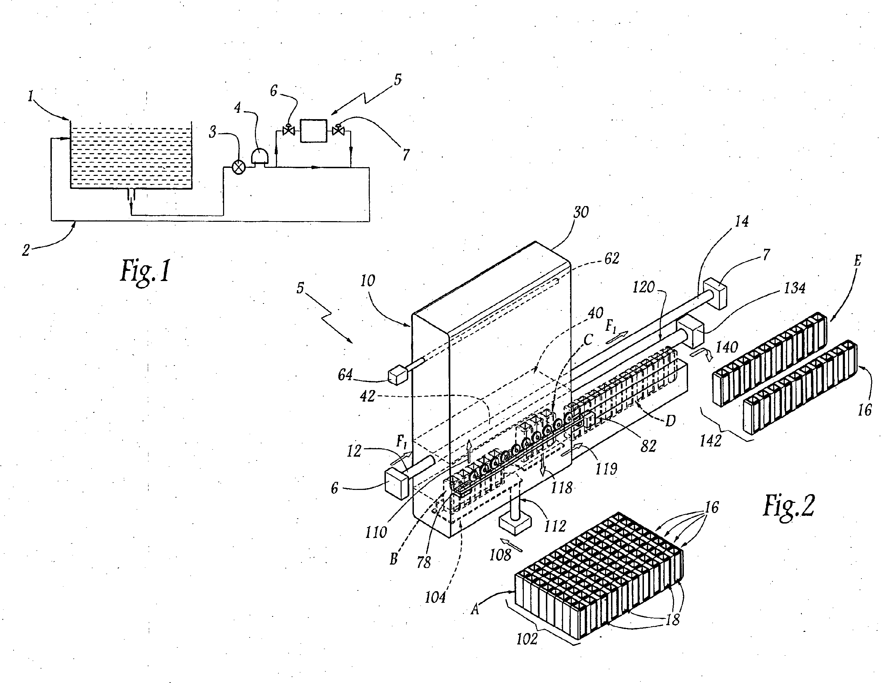

Sur la figure 1 sont représentés une piscine 1 et un circuit 2 de filtration de l'eau contenue dans la piscine. Le circuit 2 est fermé sur la piscine 1 : par exemple, l'eau alimentant le circuit provient du fond de la piscine et est renvoyée par le circuit dans la piscine, à proximité de la surface de l'eau. Le circuit est équipé d'une pompe 3 d'entraînement de l'eau dans le circuit et d'une unité 4 de filtrage de l'eau. En aval de l'unité 4, une installation 5 de prélèvement et de test qualitatif de l'eau est raccordée en dérivation du circuit 2. Cette installation 5 est munie à son entrée et à sa sortie d'électrovannes 6 et 7.

Comme représenté plus en détail à la figure 2, l'installation 5 comporte un dispositif 10 traversé par l'eau circulant dans le circuit 2, entre les électrovannes 6 et 7. A cet effet, une conduite 12 raccorde l'électrovanne amont 6 au dispositif 10, tandis qu'une conduite 14 raccorde ce dispositif à l'électrovanne aval 7.

Au sein de l'installation 5, le dispositif 10 est associé à des barrettes 16, dont chacune est destinée à occuper successivement différentes configurations d'utilisation par rapport au dispositif 10, qui sont respectivement visées par des références A à E sur la figure 2 et qui vont être détaillées ci-après.

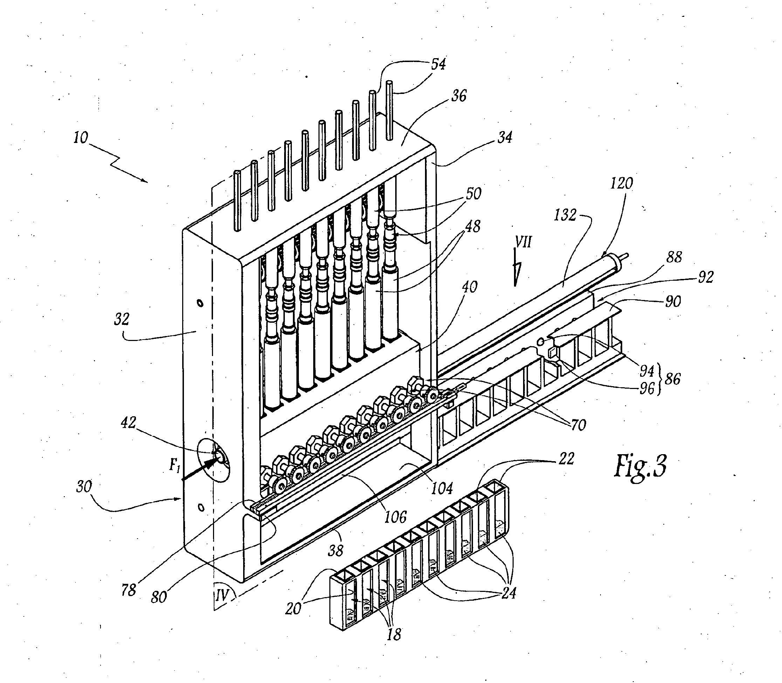

Comme représenté plus en détail à la figure 3, chaque barrette 16 présente une forme globalement parallélépipédique, destinée à s'étendre horizontalement en service. Suivant sa longueur, la barrette 16 délimite successivement dix compartiments internes 18. Suivant une direction horizontale perpendiculaire à la direction longitudinale de la barrette, chaque compartiment 18 est fermé par des parois en vis-à-vis 20, réalisées en un matériau transparent ou semi-transparent. Selon la verticale, chaque compartiment est fermé vers le haut par une paroi 22 moins résistante que les autres parois délimitant le compartiment, la paroi 22 étant ainsi prévue déchirable ou perforable.

En pratique, chaque compartiment 18 peut être délimité par un bac élémentaire de petites dimensions, comportant, entre autres, les parois 20 et 22, de sorte que dix de ces petits bacs sont agencés dans un cadre d'assemblage, conformé de manière dédiée, pour former la barrette 16. D'autres formes de réalisation de la barrette sont bien entendues envisageables.

Un réactif 24 est placé dans chaque compartiment 18, en reposant sur la paroi de fond de ce compartiment. Ce réactif se présente sous des formes diverses, et ce de manière potentiellement différente pour chacun des compartiments. Ainsi, dans l'exemple considéré à la figure 3, les sept premiers compartiments dirigés vers le lecteur contiennent des réactifs respectifs 24 sous forme d'une poudre ou de granulés, tandis que les trois compartiments restants contiennent des réactifs respectifs 24 sous forme d'un liquide.

Comme représenté à la figure 3, le dispositif 10 comporte un châssis rigide 30 comprenant deux montants verticaux 32 et 34, reliés à leurs extrémités supérieure et inférieure par des traverses horizontales 36 et 38. Par commodité, la suite de la description est orientée par rapport au châssis 30, de sorte que le terme « avant » désigne une direction horizontale dirigée vers le montant 32, tandis que le terme « arrière » désigne une direction de sens opposé.

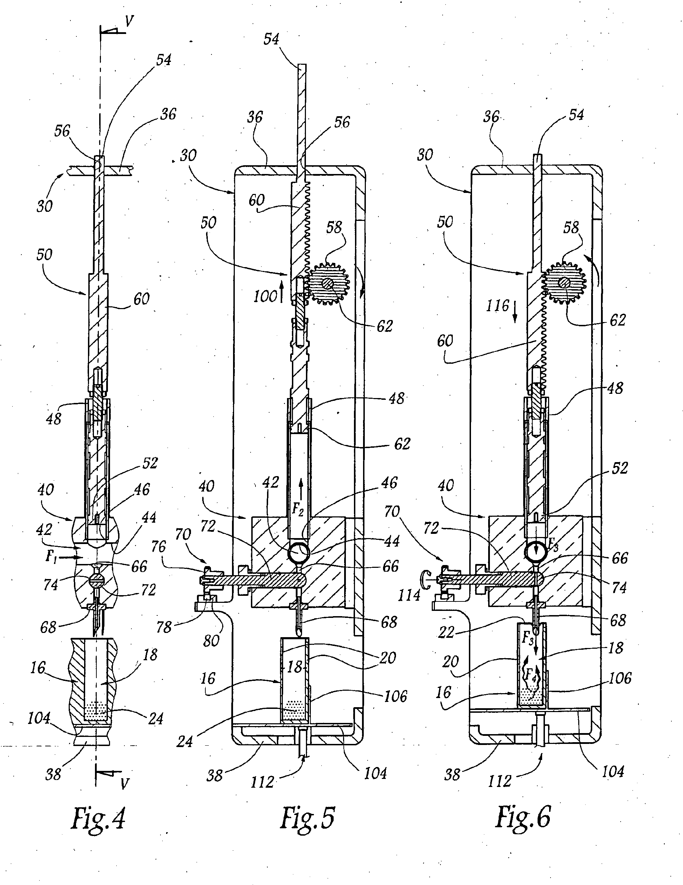

Entre les montants avant 32 et arrière 34, le dispositif 10 comprend un corps 40 globalement parallélépipédique, assemblé de manière fixe au châssis 30 et traversé de part en part, suivant sa longueur, par une voie de circulation d'eau 42 qui relie les conduites 12 et 14. Comme vu en coupe sur les figures 4 et 5, le corps 40 délimite un canal interne longitudinal 44 dont les extrémités longitudinales débouchent dans des ouvertures traversantes des montants 32 et 34, pour être respectivement raccordées aux extrémités aval et amont des conduites 12 et 14.

Suivant sa longueur, le corps 40 délimite successivement dix passages verticaux 46, reliant le canal 44 à la face supérieure du corps 40, comme représenté aux figures 4 et 5. Le débouché supérieur de chacun des passages 46 est prolongé verticalement vers le haut par un cylindre co-axial 48 solidaire du corps 40. A l'intérieur de chaque cylindre 48 est monté un piston 50 déplaçable en translation verticale par rapport au cylindre et donc par rapport au corps 40 et au châssis 30. Chaque piston 50 comporte, à son extrémité inférieure, une tête 52 à même de coulisser de manière étanche contre la face intérieure du cylindre correspondant 48, tandis qu'à son extrémité supérieure, chaque piston est constitué d'une tige terminale verticale 54, montée coulissante, de manière ajustée, dans un trou traversant 56 de la traverse supérieure 36. De la sorte, en service, chaque piston 50 est à même d'être translaté vers le haut et vers le bas par rapport au châssis 30, en étant efficacement guidé par la coopération de sa tige terminale 54 et du trou traversant 56 correspondant.

Les déplacements de chaque piston 50 sont commandés par une roue dentée 58 à axe rotatif horizontal, en prise avec la partie courante 60 du piston, qui forme une crémaillère. Les dix roues dentées 58 sont portées par un arbre commun 62 porté à l'horizontale par le châssis 30, les extrémités avant et arrière de cet arbre étant supportées par les montants 32 et 34, notamment avec interposition de roulements non visibles sur les figures. L'entraînement de l'arbre 62 est assuré par un moteur électrique 64, représenté de manière schématique uniquement à la figure 2 et, par exemple, agencé à l'extrémité avant de cet arbre. En pratique, le carter du moteur 64 est avantageusement supporté par le châssis 30.

En revenant aux figures 4 et 5, on voit que, suivant sa longueur, le corps 40 délimite successivement dix passages verticaux 66 reliant le canal 44 à la face inférieure du corps 40. Chacun de ces passages 66 s'étend dans le prolongement vertical d'un des passages 46, le canal 44 séparant ainsi les deux passages 46 et 66 en regard l'un de l'autre.

Le débouché inférieur de chaque passage 66 est muni d'un perforateur 68, sous forme d'une pointe biseautée creuse dirigée vers le bas, reliant intérieurement le passage 66 à l'extérieur du corps 40.

Chaque passage 66 est associé à un robinet d'obturation 70 dont la tige 72 s'étend horizontalement à l'intérieur du corps 40, entre la partie courante du passage 66 et l'une des faces latérales du corps 40. A son extrémité tournée vers le passage 66, la tige 72 commande l'écoulement à travers ce passage et délimite à cet effet un trou traversant 74 s'étendant suivant un diamètre de la tige : de cette façon, selon la position angulaire de la tige 72 autour de son axe longitudinal central, le trou 74 permet ou non la communication fluidique entre les parties d'extrémité supérieure et inférieure du passage 66.

Pour permettre la commande de la position angulaire de la tige 72, chaque robinet d'obturation 70 comporte, à l'extrémité de la tige 72 opposée au passage 66, une roue dentée 76 solidaire de la tige. Les dix roues 76 sont en prise avec une crémaillère horizontale commune 78, montée à coulissement horizontal dans un guide 80 solidaire du châssis 30. L'entraînement de la crémaillère 78 est assuré par un moteur électrique 82, représenté de manière schématique uniquement à la figure 2 et agencé, par exemple, à l'extrémité arrière du guide 80. En pratique, le carter du moteur 82 est avantageusement supporté par le châssis 30.

Le dispositif 10 comporte en outre des moyens 86 de mesure de caractéristiques physico-chimiques d'une solution aqueuse. Ces moyens 86 sont portés par deux barres horizontales 88 et 90 appartenant au châssis 30 et s'étendant vers l'arrière du montant arrière 34, en étant solidarisé fixement à ce montant, par exemple par soudage. Les barres 88 et 90 s'étendent en regard l'une de l'autre à un même niveau vertical, en ménageant entre elles un espace libre 92 destiné à recevoir, de manière sensiblement complémentaire, une des barrettes 16, comme expliqué plus loin. A son extrémité avant, cet espace libre 92 débouche dans le volume délimité entre les montants 32 et 34 et les traverses 36 et 38, au-dessous du corps 40.

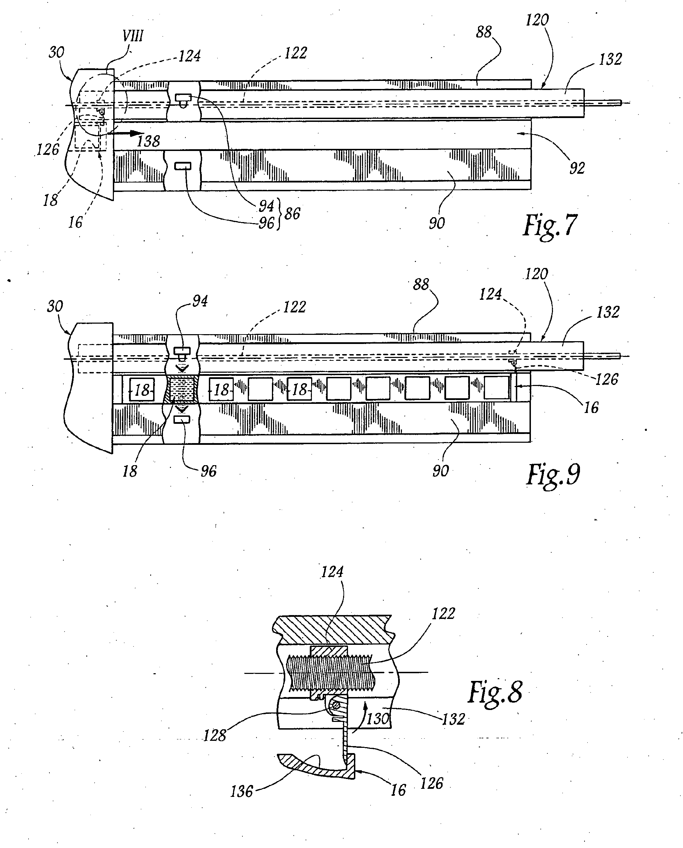

Les moyens de mesure 86 comportent dix diodes 94 portées par la barre 88, en étant régulièrement réparties le long de cette barre. Comme représenté de manière schématique aux figures 7 et 9 pour l'une de ces diodes, ces dernières émettent chacune un rayon lumineux dirigé vers la barre 90 et destiné à être capté par des photodétecteurs respectifs 96 portés par cette barre 90.

L'installation 5 et le dispositif 10 comportent d'autres composants, qui vont être présentés ci-après dans le cadre de la description du fonctionnement de cette installation et de ce dispositif.

En vue de tester la qualité de l'eau contenue dans la piscine 1, la pompe 3 est actionnée de manière à faire circuler de l'eau dans le circuit 2, au travers de l'unité de filtrage 4. Il est recommandé de maintenir la circulation d'eau dans le circuit 2 en période diurne, ainsi que d'attendre un laps de temps suffisant avant d'envoyer l'eau au travers du dispositif 10 pour que cette eau soit représentative de l'eau contenue dans la piscine.

En ouvrant les électrovannes 6 et 7, de l'eau est dérivée du circuit 2 pour traverser le dispositif 10 en formant la voie de circulation 42, via les conduites 12 et 14, comme indiqué par les flèches F₁ sur les figures. Cette eau circule ainsi dans le canal 44.

Le moteur 64 est ensuite actionné, de manière à ce que l'arbre 62 entraîne les pistons 50 en translation vers le haut, comme indiqué par la flèche 100 à la figure 5 : là tête 52 de chaque piston coulisse vers le haut à l'intérieur du cylindre correspondant 48 et aspire, à l'intérieur de ce cylindre, de l'eau depuis le canal 44, comme indiqué par la flèche F₂.

Une fois que les pistons 50 ont atteint leur hauteur maximale, les électrovannes 6 et 7 sont fermées, l'eau prélevée depuis la voie de circulation 42 demeurant ainsi à l'intérieur des cylindres 48.

Avant, pendant ou après la translation vers le haut des pistons 50, une des barrettes 16 est déplacée par rapport au dispositif 10, de manière à passer de sa configuration A à sa configuration B : dans leur configuration A, plusieurs barrettes 16 sont accolées les une aux autres, de manière à constituer un stock 102 de barrette non encore utilisées par le dispositif. Au moyen d'un chargeur automatique non représenté, une des barrettes du stock 102 est introduite à l'horizontale entre les montants 32 et 34, au-dessous du corps 40, jusqu'à occuper la position de la configuration B, comme indiqué par la flèche 108 sur la figure 2. En pratique, la barrette en configuration B repose sur une plaque horizontale de semelle 104, agencée entre les montants 32 et 34, de manière verticalement déplaçable vis-à-vis de la traverse 38. Pour faciliter le positionnement de la barrette dans la configuration B, la plaque de semelle 104 est munie d'une butée 106 s'opposant au déplacement de la barrette au delà de sa position de la configuration B, dans le sens de la flèche 108.

La barrette 16 passe ensuite de sa configuration B à sa configuration C, par translation verticale vers le haut, comme indiqué par la flèche 110. A cet effet, le dispositif 10 est muni d'un actionneur 112 de soulèvement de la plaque de semelle 104, tel qu'un vérin électrique, représenté de manière schématique uniquement à la figure 2. Le déplacement de la barrette 16 entre les positions des configurations B et C est également illustré aux figures 5 et 6 : ce déplacement provoque la perforation des parois supérieures 22 des compartiments 18, par les perforateurs 68.

Alors que la barrette 16 est dans sa configuration C, le moteur 82 est actionné de manière à ouvrir les robinets d'obturation 70, par entraînement rotatif des tiges 72, comme indiqué par la flèche 114 à la figure 6. Le moteur 64 est ensuite de nouveau actionné, de manière que l'arbre 62 descende les pistons 50, comme indiqué par la flèche 116 à la figure 6 : l'eau, qui a été prélevée depuis la voie de circulation 42 et stockée dans les cylindres 48, est alors injectée, sous la pression des têtes de piston 52, dans les compartiments 18 de la barrette 16, en circulant dans les passages 66, les trous 74 et les perforateurs 68, comme indiqué par les flèches F₃ à la figure 6. Cette eau se mélange alors avec les réactifs 24 situés au fond de chacun des compartiments 18, pour former une solution aqueuse contenue dans le compartiment, comme indiqué par les flèches F₄ à la figure 6. La pression d'injection de l'eau provoque des turbulences dans les compartiments, ce qui homogénise les solutions aqueuses.

La configuration C correspond ainsi à une configuration de remplissage des compartiments 18 de la barrette 16, tandis que la configuration B correspond à une position de repos pour la barrette, dans le sens où ses compartiments sont fluidiquement déconnectés du corps 40, en demeurant à distance de ce dernier.

Une fois que le mélange des réactifs 24 et de l'eau provenant des cylindres 48 est réalisé dans les compartiments 18 de la barrette 16, cette dernière est replacée de sa configuration C à sa configuration B, comme indiqué par la flèche 118. A cette fin, l'actionneur 112 déplace la plaque de semelle 104 suivant un mouvement de translation vers le bas, opposé au mouvement de translation de la flèche 110. Avant de redescendre la plaque de semelle 104, les robinets 70 sont manoeuvrés par actionnement du moteur 82, de manière à obturer les passages 66.

Une fois de retour à sa configuration B, la barrette 16 est déplacée, comme indiqué par la flèche 119, jusqu'à la configuration D, dans laquelle la barrette est logée dans l'espace libre 92 séparant les barres 88 et 90. A cet effet, le dispositif 10 comporte un mécanisme 120 de déplacement rectiligne vers l'arrière de la barrette 16 : comme représenté plus en détail sur les figures 7 à 9, ce mécanisme, qui fonctionne à la façon d'un système vis-écrou, comporte une tige horizontale filetée 122 autour de laquelle est rapporté de manière mobile un écrou 124. Le corps de cet écrou est muni d'un doigt 126 rabattable contre le corps de l'écrou, en basculant autour d'un axe vertical 128. Le doigt 126 est normalement maintenu par un ressort, non représenté, dans une position déployée par rapport au corps de l'écrou, illustrée à plus grande échelle à la figure 8, dans laquelle le doigt s'étend en longueur suivant une direction radiale à la tige 122, tandis que sous l'action d'une résistance orthoradiale à l'axe 128, le ressort précité cède et le doigt 126 se rabat vers la tige, comme indiqué par la flèche 130 à la figure 8.

Le mécanisme 120 comporte également un cylindre horizontal externe 132, centré autour de la tige 122 et fendu sur toute sa longueur, avec le doigt 26 reçu entre les bords de la fente correspondante. De la sorte, lorsque la tige 122 est entraînée en rotation sur elle-même au moyen d'un moteur électrique 134, représenté de manière schématique uniquement à la figure 2 et agencé, par exemple, à l'extrémité arrière de la tige 122, l'écrou 124 se translate le long de la tige, son doigt 126 le bloquant en rotation.

En pratique, le cylindre 132 est porté par le châssis 30, son extrémité avant étant notamment reçue et immobilisée, par exemple par emmanchement, dans une ouverture correspondante du montant arrière 34, tandis que des roulements, non représentés, sont interposés entre la tige 122 et le cylindre. De même, le carter du moteur 134 est avantageusement supporté par le châssis 30, notamment par les barres 88 et 90.

Pour déplacer la barrette 16 de sa position de la configuration B à sa position de la configuration D, l'écrou 124 est positionné à l'extrémité avant de la tige 122, de sorte que son doigt 126 vient en prise dans une encoche correspondante 136 délimitée dans une paroi associée de la barrette 16, comme représenté aux figures 7 et 8. Par actionnement du moteur 134, l'écrou 124 se translate vers l'arrière le long de la tige 122, comme indiqué par la flèche 138 à la figure 7, jusqu'à la proximité arrière de cette tige. La barrette 16 est alors dans sa configuration D, dans laquelle chacun de ses compartiments 18 est horizontalement interposé entre l'un des couples diode 94/photodétecteur 96, comme visible pour l'un de ces couples à la figure 9. Chaque diode est alors allumée et son rayonnement atteint le photodétecteur correspondant après avoir successivement traversé les parois 20 du compartiment correspondant 18 et la solution aqueuse contenue dans ce compartiment. Le photodétecteur 96 mesure l'intensité lumineuse provenant de la diode 94 et fournit un signal représentatif de cette mesure à une unité de traitement, non représentée, à même de calculer une ou plusieurs caractéristiques physico-chimiques de la solution aqueuse, telles que son alcalinité. Sur la base de ces résultats de mesure, fournis à l'utilisateur, ce dernier décide, éventuellement avec une assistance électronique, d'éventuelles actions correctrices de la qualité de l'eau de la piscine 1.

La configuration D correspond ainsi à une configuration de test qualitatif des solutions aqueuses contenues dans les compartiments 18.

On comprend que pour limiter d'éventuelles interférences lumineuses entre deux compartiments 18 adjacents lors de l'allumage de leur diode associée 94, les montants verticaux de la barrette 16 séparant les parois 20 les unes des autres sont avantageusement opaques, en particulier pour les longueurs d'onde des rayonnements émis par les diodes. De même, pour ne pas perturber la réception des photodétecteurs 96, des plaques de fermeture ou analogue sont avantageusement rapportées autour des barres 88 et 90.

Une fois que toutes les mesures relatives aux solutions aqueuses ont été effectuées par les moyens de mesure 86, la barrette 16 passe de sa configuration D à sa configuration E, par éjection de la barrette depuis les barres 88 et 90, comme indiqué par la flèche 140 à la figure 2. En pratique, l'éjection de la barrette est réalisée par un éjecteur automatique, non représenté sur les figures, qui dégage du dispositif 10 la barrette jusque dans sa configuration E, notamment en lui faisant rejoindre un stock 142 d'autres barrettes usagées.

Ainsi, le fonctionnement du dispositif 10 est totalement automatisé : en service, aucune intervention humaine n'est nécessaire pour assurer un fonctionnement correct, sous réserve que des barrettes non utilisées 16 soient à disposition du dispositif 10 et que de l'électricité soit fournie aux moteurs 64, 82 et 134 ainsi qu'à l'actionneur 112. En pratique, une unité de supervision, non représentée, est prévue pour coordonner l'actionnement des vannes 6 et 7, des moteurs 64, 82 et 134, de l'actionneur 112, du chargeur et de l'éjecteur. En plus du confort d'utilisation qu'elle procure, cette automatisation garantit une bonne répétabilité des mesures, ainsi qu'un dosage précis en ce qui concerne l'eau prélevée par les pistons 50. A ce titre, en variante non représentée, chacun des pistons 50 peut présenter des aménagements propres, notamment en ce qui concerne sa partie courante 60 formant crémaillère, de manière que les courses respectives des différents pistons soient différentes les unes des autres, conduisant ainsi à des prélèvements de quantités d'eau différentes dans chacun des cylindres 48 du dispositif. Lors de l'injection de ces prélèvements dans les différents compartiments 18 de la barrette 16 en configuration C, des quantités dosées d'eau différentes sont alors introduites dans les compartiments 18, si besoin en agençant dans le canal 44 des moyens d'obturation empêchant la circulation d'eau par ce canal entre les différents passages 66.

Par ailleurs, la structure du dispositif 10 est robuste : le châssis 30, qui porte avantageusement tous les autres composants du dispositif, permet d'installer rapidement et aisément le dispositif et l'utilisation de mouvements rectilignes pour faire passer la barrette entre les configurations B, C et D garantit une bonne fiabilité.

Divers aménagements et variantes au dispositif 10 et à l'installation 5 décrits ci-dessus sont en outre envisageables :
- le nombre de compartiments 18 peut être supérieur ou inférieur à dix, le nombre de pistons 50 de remplissage de ces compartiments et le nombre de couples diode 94/photodétecteur 96 étant modifiés de manière correspondante ;
- d'autres formes de motorisation que les moteurs électriques 64, 82 et 134 et que l'actionneur électrique 112 peuvent être utilisées, du moment qu'elles permettent l'entraînement de la barrette 16 entre les différentes configurations B à D, ainsi que la commande des prélèvements d'eau depuis la voie de circulation 42 et des remplissages des compartiments 18 de la barrette ; et/ou
- la barrette 16 peut occuper des configurations intermédiaires entre les configurations de repos B, de remplissage C et de test qualificatif D ; ainsi, à titre d'exemple, l'un des compartiments 18 peut, avant d'être chargé dans le dispositif 10, être rempli d'une solution neutre, telle que de l'eau distillée : dans la configuration de remplissage C, ce compartiment est laissé intact, puis, après avoir déplacé la barrette par le mécanisme 120 dans une position intermédiaire, cette solution neutre est aspirée du compartiment précité, par un moyen ad hoc, pour être introduite dans un autre des compartiments 18, notamment afin de diluer de manière contrôlée la solution aqueuse formée dans cet autre compartiment par son réactif et de l'eau prélevée provenant de la piscine.

## Revendications

1. Dispositif (10) de prélèvement et de test qualitatif de l'eau d'un bassin (1), tel qu'une piscine, comportant:
- une barrette (16) délimitant au moins un compartiment (18) contenant un réactif (24) destiné à être mélangé dans le compartiment avec de l'eau provenant du bassin, pour former une solution aqueuse,
- des moyens automatisés (40, 50, 62, 64, 70, 78, 82) de remplissage du ou de chaque compartiment par de l'eau prélevée depuis une voie (42) de circulation d'eau provenant du bassin, et
- des moyens (86) de mesure d'au moins une caractéristique physico-chimique de la solution aqueuse,
**caractérisé en ce qu'**il comporte en outre des moyens (112, 120) d'entraînement motorisé de la barrette (16) par rapport à un châssis (10) de support des moyens de remplissage (40, 50, 62, 64, 70, 78, 82) et des moyens de mesure (86), depuis une configuration de repos (configuration B), dans laquelle le ou chaque compartiment est déconnecté des moyens de remplissage, à une configuration de test qualitatif (configuration D), dans laquelle, au niveau du ou de chaque compartiment, la solution aqueuse, formée par le mélange du réactif et de l'eau fournie par les moyens de remplissage, est soumise aux moyens de mesure, lesquels moyens d'entraînement comportent, d'une part, des premiers moyens (112) de déplacement réversible de la barrette (16) par rapport au châssis (10) entre une position associée à la configuration de repos (configuration B) et une position de remplissage, dans laquelle les moyens de remplissage (40, 50, 62, 64, 70, 78, 82) sont raccordés à le ou chaque compartiment (18) et envoient l'eau prélevée dans le compartiment (configuration C), et, d'autre part, des seconds moyens (120) de déplacement de la barrette depuis sa position associée à la configuration de repos à une position associée à la configuration de test qualitatif (configuration D).

2. Dispositif suivant la revendication 1, **caractérisé en ce que** les premier et second moyens de déplacement (112, 120) sont adaptés pour déplacer la barrette (16) suivant des mouvements respectifs sensiblement rectilignes (flèches 110, 118 et 119).

3. Dispositif suivant l'une quelconque des revendications précédentes, **caractérisé en ce que** les moyens de remplissage comportent, pour le ou chaque compartiment (18), un piston (50) mobile par rapport au châssis (10), adapté, à la fois, pour aspirer (F₂) de l'eau depuis la voie de circulation (42) et pour injecter (F₃) l'eau ainsi aspirée vers le compartiment.

4. Dispositif suivant la revendication 3, **caractérisé en ce que** le ou les pistons (50) sont commandés en déplacement par un actionneur motorisé (62, 64), avantageusement porté par le châssis (30).

5. Dispositif suivant l'une quelconque des revendications précédentes, **caractérisé en ce que** les moyens de remplissage comportent un corps (40) solidaire du châssis (30), qui est traversé par la voie de circulation (42) et qui, pour le ou chaque compartiment (18), délimite à la fois un passage (46) de prélèvement d'eau provenant de la voie de circulation et un passage obturable (66) d'écoulement d'eau depuis le passage de prélèvement vers le compartiment.

6. Dispositif suivant la revendication 5, **caractérisé en ce que** les passages de prélèvement (46) et d'écoulement (66) s'étendent transversalement à la voie de circulation (42), dans le prolongement l'un de l'autre, en étant séparés par cette voie de circulation.

7. Dispositif suivant l'une des revendications 5 ou 6, **caractérisé en ce que** le ou chaque passage d'écoulement (66) est muni d'un obturateur (70) mobile par rapport au corps (40) et commandé en déplacement par un actionneur motorisé (78, 82), avantageusement porté par le châssis (30).

8. Dispositif suivant l'une quelconque des revendications 5 à 7, **caractérisé en ce que** le corps (40) est muni, au niveau du débouché du ou de chaque passage d'écoulement (66) vers le compartiment correspondant (18), d'un moyen de perforation (68) adapté pour perforer une paroi (22) du compartiment lorsque la barrette (16) est déplacée depuis sa configuration de repos (configuration B) vers le corps.

9. Installation de prélèvement et de test qualitatif de l'eau d'un bassin (1), tel qu'une piscine,
**caractérisée en ce qu'**elle comporte :
- un dispositif de prélèvement et de test qualitatif (10) conforme à l'une quelconque des revendications précédentes,
- au moins une vanne (6, 7) de commande de la circulation de l'eau provenant du bassin (1) dans la voie de circulation (42),
- un chargeur automatique adapté pour mettre en place (flèche 108) dans le dispositif (10) une barrette (16) en configuration de repos (configuration B), provenant d'un stock (102) de barrettes non encore utilisées, et
- un éjecteur automatique adapté pour dégager (flèche 140) du dispositif (10) la barrette en configuration de test qualitatif (configuration D).

## Patentansprüche

1. Vorrichtung (10) zur Entnahme und Qualitätsprüfung von Wasser eines Bassins (1), wie eines Schwimmbads, umfassend:
- eine Leiste (16), die mindestens ein Fach (18) begrenzt, das ein Reagenz (24) enthält, das vorgesehen ist, in dem Fach mit aus dem Bassin stammenden Wasser gemischt zu werden, um eine wässrige Lösung zu bilden,
- automatisierte Mittel (40, 50, 62, 64, 70, 78, 82) zum Füllen des oder jedes Fachs mit Wasser, das aus einem Zirkulationsweg (42) für aus dem Bassin stammenden Wasser entnommen wird, und
- Mittel (86) zum Messen mindestens eines physikalisch-chemischen Parameters der wässrigen Lösung,
**dadurch gekennzeichnet, dass** sie außerdem Mittel (112, 120) zum motorisierten Transport der Leiste (16) in Bezug auf ein Gestell (10) zum Lagern der Mittel (40, 50, 62, 64, 70, 78, 82) zum Füllen und der Mittel (86) zum Messen aus einer Ruhestellung (Stellung B), in der das oder jedes Fach von den Mitteln zum Füllen abgekoppelt ist, in eine Stellung der Qualitätsprüfung (Stellung D), in der die wässrige Lösung in dem oder jedem Fach, die durch die Mischung des Reagenz mit dem von den Mitteln zum Füllen gelieferten Wasser gebildet wird, den Mitteln zum Messen unterworfen wird, wobei die Mittel zum Transport einerseits erste Mittel (112) zum reversiblen Verschieben der Leiste (16) in Bezug auf das Gestell (10) zwischen einer der Ruhestellung (Stellung B) zugeordneten Position und einer Füllposition, in der die Mittel (40, 50, 62, 64, 70, 78, 82) zum Füllen an das oder jedes Fach (18) angeschlossen sind und das entnommene Wasser in das Fach leiten (Stellung C), und andererseits zweite Mittel (120) zum Verschieben der Leiste aus ihrer der Ruhestellung zugeordneten Position in eine Position, die der Stellung der Qualitätsprüfung (Stellung D) zugeordnet ist, aufweisen.

2. Vorrichtung nach Anspruch 1, **dadurch gekennzeichnet, dass** die ersten und zweiten Mittel (112, 120) zum Verschieben angepasst sind, die Leiste (16) jeweils gemäß im Wesentlichen geradlinigen Bewegungen (Pfeile 110, 118 und 119) zu bewegen.

3. Vorrichtung nach einem beliebigen der vorhergehenden Ansprüche, **dadurch gekennzeichnet, dass** die Mittel zum Füllen für das oder jedes Fach (18) einen in Bezug auf das Gestell (10) beweglichen Kolben (50) aufweisen, der angepasst ist, gleichzeitig Wasser aus dem Zirkulationsweg (42) anzusaugen (F₂) und das so angesaugte Wasser in das Fach einzuleiten (F₃).

4. Vorrichtung nach Anspruch 3, **dadurch gekennzeichnet, dass** der oder die Kolben (50) hinsichtlich der Verschiebung durch ein motorisiertes Betätigungselement (62, 64), das vorteilhafterweise von dem Gestell (30) getragen wird, gesteuert werden.

5. Vorrichtung nach einem beliebigen der vorhergehenden Ansprüche, **dadurch gekennzeichnet, dass** die Mittel zum Füllen einen mit dem Gestell (30) verbunden Körper (40) aufweisen, der von dem Zirkulationsweg (42) durchquert wird und der für das oder jedes Fach (18) sowohl einen Kanal (46) zur Entnahme von aus dem Zirkulationsweg stammenden Wasser als auch einen verschließbaren Kanal (66) zum Ableiten von Wasser aus dem Kanal zur Entnahme zu dem Fach begrenzt.

6. Vorrichtung nach Anspruch 5, **dadurch gekennzeichnet, dass** die Kanäle (46, 66) zur Entnahme und zum Ableiten sich in der Verlängerung des einen zum anderen quer zum Zirkulationsweg (42) erstrecken, wobei sie von diesem Zirkulationsweg getrennt sind.

7. Vorrichtung nach einem der Ansprüche 5 oder 6, **dadurch gekennzeichnet, dass** der oder jeder Kanal (66) zum Ableiten mit einem zum Körper (40) beweglichen Verschlusselement (70) ausgerüstet ist, das hinsichtlich der Bewegung durch ein motorisiertes Betätigungselement (78, 82) gesteuert wird, das vorteilhafterweise an dem Gestell (30) gelagert ist.

8. Vorrichtung nach einem beliebigen der Ansprüche 5 bis 7, **dadurch gekennzeichnet, dass** der Körper (40) an der Mündung des oder jedes Kanals (66) zum Ableiten in das entsprechende Fach (18) mit einem Perforationsmittel (68) ausgerüstet ist, das angepasst ist, eine Wand (22) des Fachs zu perforieren, wenn die Leiste (16) aus ihrer Ruhestellung (Stellung B) zu dem Körper verschoben wird.

9. Anlage zur Entnahme und zur Qualitätsprüfung von Wasser eines Bassins (1), wie eines Schwimmbads,
**dadurch gekennzeichnet, dass** sie umfasst:
- eine Vorrichtung (10) zur Entnahme und zur Qualitätsprüfung entsprechend einem beliebigen der vorhergehenden Ansprüche,
- mindestens ein Ventil (6, 7) zur Steuerung der Strömung des aus dem Bassin (1) stammenden Wassers in den Zirkulationsweg (42),
- eine automatische Ladevorrichtung, die angepasst ist, eine Leiste (16) in eine Ruhestellung (Stellung B) in der Vorrichtung (10) in Stellung zu bringen (Pfeil 108), wobei die Leiste von einem Lagerbestand (102) von noch nicht verwendeten Leisten herkommen, und
- einen automatischen Auswerfer, der angepasst ist, die Leiste in der Stellung der Qualitätsprüfung (Stellung D) aus der Vorrichtung (10) freizugeben (Pfeil 140).

## Claims

1. Device (10) for sampling and qualitatively testing the water in a basin (1), such as a swimming pool, comprising:
- a bar (16) delimiting at least one compartment (18) which contains a reagent (24) intended to be mixed in the compartment with water which comes from the basin, in order to form an aqueous solution,
- automated means (40, 50, 62, 64, 70, 78, 82) for filling the one or each compartment with water drawn from a water circulation path (42) which comes from the basin, and
- means (86) for measuring at least one physico-chemical characteristic of the aqueous solution,
**characterised in that** it comprises in addition means (112, 120) for motorised actuation of the bar (16) relative to a frame (10) for supporting filling means (40, 50, 62, 64, 70, 78, 82) and measuring means (86), from an inoperative configuration (configuration B), in which the one or each compartment is disconnected from the filling means, to a qualitatively testing configuration (configuration D) in which, at the level of the one or each compartment, the aqueous solution which is formed by the mixture of the reagent and the water provided by the filling means is subjected to measuring means, which actuation means comprise, on the one hand, first means (112) for reversible displacement of the bar (16) relative to the frame (10) between a position connected to the inoperative configuration (configuration B) and a filling position in which the filling means (40, 50, 62, 64, 70, 78, 82) are connected to the one or each compartment (18) and send the sample water into the compartment (configuration C) and, on the other hand, second means (120) for displacing the bar from its position connected to the inoperative configuration to a position connected to the qualitatively testing configuration (configuration D).

2. Device according to claim 1, **characterised in that** the first and second displacement means (112, 120) are adapted to displace the bar (16) according to substantially rectilinear respective movements (arrows 110, 118 and 119).

3. Device according to any of the preceding claims, **characterised in that** the filling means comprise, for the one or each compartment (18), a piston (50) which is moveable relative to the frame (10), and is adapted, at the same time, to draw in (F₂) water from the circulation path (42) and to inject (F₃) the thus drawn-in water towards the compartment.

4. Device according to claim 3, **characterised in that** the piston or pistons (50) are controlled, with respect to displacement, by a motorised actuator (62, 64) which is advantageously carried by the frame (30).

5. Device according to any of the preceding claims, **characterised in that** the filling means comprise a body (40) which is integral with the frame (30) and is traversed by the circulation path (42) and which, for the one or each compartment (18), delimits, at the same time, a passage (46) for sample water which comes from the circulation path and a sealable passage (66) for water flowing from the sample passage towards the compartment.

6. Device according to claim 5, **characterised in that** the sample (46) and flow (66) passages extend transversely to the circulation path (42), in the extension of one from the other, being separated by this circulation path.

7. Device according to one of the claims 5 or 6, **characterised in that** the one or each flow passage (66) is provided with a seal (70) which is moveabe relative to the body (40) and is controlled, with respect to displacement, by a motorised actuator (78, 82) which is advantageously carried by the frame (30).

8. Device according to any of the claims 5 to 7, **characterised in that** the body (40) is provided, at the level of the outlet from the one or each flow passage (66) towards the corresponding compartment (18), with a perforation means (68) which is adapted to perforate a wall (22) of the compartment when the bar (16) is displaced from its inoperative configuration (configuration B) towards the body.

9. Facility for sampling and qualitatively testing the water in a basin (1), such as a swimming pool, **characterised in that** it comprises:
- a device for sampling and qualitatively testing (10) according to any of the preceding claims,
- at least one valve (6, 7) for controlling the circulation of water coming from the basin (1) in the circulation path (42),
- an automatic loader adapted to position (arrow 108) in the device (10) a bar (16) in an inoperative configuration (configuration B), coming from a store (102) of bars which have not yet been used, and
- an automatic ejector adapted to release (arrow 140) the bar in the qualitatively testing configuration (configuration D) from the device (10).
